# EUROPÄISCHE PATENTSCHRIFT

(11) **EP 3 019 212 B1**
(45) Veröffentlichungstag und Bekanntmachung des Hinweises auf die Patenterteilung: **02.12.2020**
(21) Anmeldenummer: 14738389.7
(22) Anmeldetag: 07.07.2014
(51) Int. Cl.: A61M 1/36, A61M 1/10

(54) **VORRICHTUNG ZUR ÜBERWACHUNG EINER EXTRAKORPORALEN BLUTBEHANDLUNG**
DEVICE FOR MONITORING AN EXTRACORPOREAL BLOOD TREATMENT
DISPOSITIF DE SURVEILLANCE D'UN TRAITEMENT SANGUIN PAR CIRCULATION EXTRACORPORELLE

(30) Priorität: 09.07.2013 DE 102013011485; 26.07.2013 DE 102013012504
(43) Veröffentlichungstag der Anmeldung: 18.05.2016
(73) Patentinhaber: Fresenius Medical Care Deutschland GmbH, 61352 Bad Homburg (DE)
(72) Erfinder: WIKTOR, Christoph, 63571 Gelnhausen (DE); HEIDE, Alexander, 65817 Eppstein (DE); PETERS, Arne, 61352 Bad Homburg (DE)
(74) Vertreter: Behr, Wolfgang
(86) Internationale Anmeldenummer: PCT/EP2014/001858
(87) Internationale Veröffentlichungsnummer: WO 2015/003795

(56) Entgegenhaltungen:
- WO-A1-03/002174
- DE-A1-102009 060 668
- US-A1- 2013 006 128

## Beschreibung

Die Erfindung betrifft eine Vorrichtung zur Überwachung einer extrakorporalen Blutbehandlungsvorrichtung nach dem Oberbegriff des Anspruchs 1 sowie eine entsprechende Blutbehandlungsvorrichtung.

Insbesondere beschäftigt sich die vorliegende Erfindung mit dem Problem der Erkennung einer venösen Nadeldiskonnektion mittels Druckpulsmessungen am extrakorporalen Blutkreislauf.

Eine unerkannte venöse Nadeldiskonnektion kann zum befürchteten Freifluss des Blutes aus der venösen Nadel in die Umgebung führen soweit der Pumpzyklus der entsprechenden Blutpumpe nicht unterbrochen wird. Falls ein solcher schwerer Störfall nicht sofort erkannt wird, kann der Patient bei üblichen Blutflüssen von 200 ml/min bis 300 ml/min innerhalb von wenigen Minuten verbluten. Während eine Diskonnektion der arteriellen Nadel aufgrund der zwangsläufig aus der Umgebung in den extrakorporalen Blutkreislauf gesaugten Umgebungsluft sofort und zuverlässig durch die stets zur Verhinderung von Luftembolien vorhandenen Luftblasendetektoren innerhalb der Blutbehandlungsvorrichtung erkannt wird, stellt die sichere Erkennung einer venösen Nadeldiskonnektion trotz vieler bekannter Lösungsansätze immer noch eine technische Herausforderung dar. Bei den bekannten Verfahren und Vorrichtungen entstehen nämlich häufig Fehlalarme aufgrund falsch interpretierter Messwerte, so dass es sehr häufig zu grundsätzlich unerwünschten Alarmsituationen und dadurch bedingt zum Abschalten der extrakorporalen Blutbehandlungsvorrichtung kommt, obwohl keine venöse Nadeldiskonnektion vorliegt.

Bereits aus der WO 97/100 13 A1 ist es bekannt, die Integrität des extrakorporalen Blutkreislaufs anhand der Ausbreitung von Druckpulsen im extrakorporalen Blutkreislauf zu überwachen. Diesbezüglich wird vorgeschlagen, dass die auszuwertenden Druckpulse entweder mittels eines Druckwellengenerators, z. B. der Blutpumpe selbst erzeugt werden können oder alternativ in dem extrakorporalen Blutkreislauf fortgepflanzte Druckpulse vom Herz des Patienten ausgewertet werden können. In der WO 97/10013 A1 sind ausschließlich peristaltische Blutpumpen, d. h. okkludierende Blutpumpen offenbart, wobei hier darauf verwiesen wird, dass es sich bei diesen okkludierenden Blutpumpen um die für die Hämodialyse üblich verwendeten Blutpumpen handelt. Die peristaltischen Blutpumpen, auch als Rollenpumpen bezeichnet, okkludieren mit den Rollen ihres Rotors ein Pumpschlauchsegment des extrakorporalen Blutkreislaufs und erzeugen so eine pulsierende Strömung mit starken Druckpulsen. Diese Druckpulse der okkludierenden Blutpumpe bilden so starke Signale aus, dass alle sonstigen Drucksignale im extrakorporalen Blutkreislauf bei einer ungefilterten Drucksignalauswertung als Rauschen untergehen. Das führt dazu, dass eine Auswertung der Druckpulse vom Herz des Patienten zum Einen sehr aufwendige Auswertemethoden bedingen und zum Anderen trotzdem fehleranfällig sind.

Außerhalb des Gebiets der Hämodialyse sind extrakorporale Blutkreisläufe mit Zentrifugalpumpen und hier insbesondere mit Impeller-Pumpen bekannt. Derartige Systeme kommen beispielsweise bei Herzoperationen zum Einsatz.

Aus der DE 10 2009 060 668 A1 ist des Weiteren eine Vorrichtung und ein Verfahren zur Erkennung einer venösen Nadeldiskonnektion an einem extrakorporalen Blutkreislauf mit Impeller-Pumpe beschrieben. Hier wird die flache Kennlinie einer solchen Impeller-Pumpe bei konstanter Drehzahl ausgenutzt, um eine sehr kleine Druckänderung aufgrund einer venösen Nadeldiskonnektion anhand der resultierenden, zuverlässig messbaren Änderung des geförderten Blutflusses zu erkennen. Dabei wird die Erkenntnis genutzt, dass Impeller-Pumpen nicht okkludierend sind und Druckpulse passieren lassen.

Impeller-Blutpumpen sind üblicherweise Bestandteil eines Disposables bzw. Blutschlauchsatzes, insbesondere auch einer Blutkassette. Der Impeller solcher Impeller-Blutpumpen ist in der Regel magnetisch gelagert, so dass er berührungsfrei angetrieben wird. Daher kommt der Impeller der Impeller-Blutpumpe lediglich mit dem geförderten Blut in reibungsbehafteten Kontakt. Die Lagerung des Impellers kann auch eine Kombination aus magnetischer, hydraulischer und/oder mechanischer Lagerung sein.

Aus der US 2013/006128 A1 ist eine Vorrichtung gemäß dem Oberbegriff von Anspruch 1 bekannt sowie eine Blutbehandlungsvorrichtung gemäß dem Oberbegriff von Anspruch 10 bekannt.

Weitere Blutbehandlungsvorrichtungen sind aus der WO 03/002174 A1 bekannt.

Aufgabe der vorliegenden Erfindung ist es, das durch die Steuerung der Überwachungsvorrichtung durchgeführte, vorbekannte Verfahren zur Überwachung einer extrakorporalen Blutbehandlungsvorrichtung derart weiterzubilden, dass eine arterielle und/oder venöse Nadeldiskonnektion in einfacher Weise sicher erkannt wird.

Erfindungsgemäß wird diese Aufgabe durch eine Vorrichtung gemäß Anspruch 1 gelöst.

Diese erfindungsgemäße Vorrichtung dient zur Überwachung einer extrakorporalen Blutbehandlungsvorrichtung mit einem extrakorporalen Blutkreislauf, der eine arterielle Blutleitung mit einem arteriellen Patientenanschluss und eine venöse Blutleitung mit einem venösen Patientenanschluss aufweist, wobei in der arteriellen oder venösen Blutleitung eine Impeller-Pumpe oder eine Zentrifugalpumpe zum Fördern von Blut im extrakorporalen Blutkreislauf angeordnet ist. Die Vorrichtung weist weiterhin eine Überwachungsvorrichtung mit einer Steuer- und Recheneinheit auf, welche programmiert und konfiguriert ist, folgende Schritte durchzuführen:
- Messen mindestens einer ersten Druckamplitude und/oder eines ersten Verlaufs einer Druckamplitude als Referenzwert an mindestens einer Druckmessstelle des extrakorporalen Blutkreislaufs,
- Berechnen mindestens eines Druckgrenzwertes aufgrund der ersten als Referenzwert aufgenommenen Druckamplitude und/oder Festlegen eines Referenz-Druckverlaufs aufgrund des ersten Verlaufs der Druckamplitude,
- Messen mindesten einer zweiten Druckamplitude und/oder eines zweiten Verlaufs der Druckamplitude an der mindestens einen Druckmessstelle des extrakorporalen Blutkreislaufs,
- Vergleichen der zweiten Druckamplitude mit dem mindestens einen Druckgrenzwert und/oder Vergleichen des zweiten Verlaufs der Druckamplitude mit dem Referenz Druckverlauf und
- Stoppen oder erhebliches Drosseln der Blutpumpe und/oder Schließen der venösen Schlauchklemme, falls eine unzulässige Abweichung von dem Referenzwert und/oder dem Referenz-Druckverlauf festgestellt wird.

Vorteilhafte Ausgestaltungen ergeben sich aus den sich an den Hauptanspruch anschließenden Unteransprüchen.

Demnach kann bei Feststellung einer unzulässigen Abweichung ein akustischer und/oder optischer und/oder taktiler Alarm ausgegeben werden, um ein schnelles Eingreifen zu ermöglichen.

In bestimmten Ausführungsformen kann anstelle des Stoppens der Blutpumpe ein erhebliches Drosseln der Blutpumpe, beispielsweise durch Absenken der Drehzahl auf weniger als 100 U/Min, bspw. ungefähr 50U/Min, vorgesehen sein.

Gemäß einer anderen vorteilhaften Ausgestaltung der Erfindung kann die Impeller-Blutpumpe oder Zentrifugal-Blutpumpe derart angesteuert sein, dass ihre Drehzahl konstant ist. In bestimmten Ausführungsformen wird die Drehzahl der Impeller-Blutpumpe oder Zentrifugal-Blutpumpe zumindest während des Messens der Druckamplituden konstant gehalten.

Des Weiteren ist es von besonderem Vorteil, dass als zu verarbeitende Druckwerte in dem erfindungsgemäßen Verfahren die Druckpulse des Herzens des Patienten aufgenommen werden.

Insbesondere kann es sich bei der erfindungsgemäßen Überwachungsvorrichtung um eine externe Überwachungsvorrichtung handeln.

Vorteilhaft weist die Vorrichtung mindestens einen Drucksensor auf.

Grundsätzlich reicht ein Drucksensor, der dabei wie die Impeller-Pumpe oder die Zentrifugalpumpe mit der Steuer- und Recheneinheit in Verbindung steht, aus.

Gemäß einer weiteren vorteilhaften Ausgestaltung der Erfindung weist die Vorrichtung die Antriebseinheit für die Impeller-Blutpumpe oder die Zentrifugalpumpe auf.

Die vorliegende Erfindung umfasst weiterhin eine Blutbehandlungsvorrichtung gemäß Anspruch 10 zum Betrieb mit einem extrakorporalen Blutkreislauf, der eine arterielle Blutleitung mit einem arteriellen Patientenanschluss und eine venöse Blutleitung mit einem venösen Patientenanschluss aufweist, wobei in der arteriellen oder venösen Blutleitung eine Impellerpumpe oder eine Zentrifugalpumpe zum Fördern von Blut im extrakorporalen Blutkreislauf angeordnet ist, mit einer Steuerung, die programmiert und konfiguriert ist, eine Nadeldiskonnektion zu erkennen, wobei die Steuerung programmiert ist, um die Drucksignale mindestens eines Drucksensors auszuwerten. Dabei ist die Steuerung so ausgeführt, dass die am extrakorporalen Blutkreislauf gemessenen Amplituden der Druckpulse vom Herz des Patienten mit mindestens einem Grenzwert oder Referenzverlauf verglichen werden, wobei die Drucksignale des mindestens einen Drucksensors bei einer korrekten Konnektion ein Summensignal aus den durch den arteriellen und den venösen Patientenanschluss zum Drucksensor gelangenden Druckpulsen vom Herz des Patienten darstellen, und wobei bereits bei einer Abweichung der Amplitude der Druckpulse von weniger als 90% und bevorzugt weniger als 75% von einer bei einer korrekten Konnektion vorliegenden Amplitude der Druckpulse auf eine Diskonnektion geschlossen wird.

Die Blutbehandlungsvorrichtung kann so ausgeführt sein, dass der Grenzwert oder Referenzverlauf aufgrund einer initialen Messung im störungsfrei betriebenen extrakorporalen Blutkreislauf zuvor festgelegt werden kann.

Die Steuer- und Recheneinheit ist bevorzugt so ausgelegt, dass der Grenzwert oder Referenzverlauf, der zuvor im Datenspeicher abgespeichert wurde, mit den aktuellen Messwerten verglichen wird, wobei beim Auftreten einer unzulässigen Abweichung der gemessenen Amplitude der Druckpulse vom Herz des Patienten auf einen fehlerhaften Gefäßzugang geschlossen wird.

Die oben beschriebene erfindungsgemäße Vorrichtung oder Blutbehandlungsvorrichtung können dabei weiter wie folgt ausgestaltet sein:
Bevorzugt wird die Amplitude der Druckpulse vom Herz des Patienten aus der Differenz der lokalen Minima und Maxima der Druckpulskurve ermittelt. Insbesondere kann die Steuerung dabei einen Minimal- und Maximalwerterfassungseinheit aufweisen und die Amplitude, welche dem Vergleich mit einem Grenzwert unterzogen wird, aus der Differenz zwischen aufeinanderfolgenden Minimal- und Maximalwerten ermitteln.

Weiterhin kann vorgesehen sein, dass die Drucksignale des mindestens einen Drucksensors bei einer korrekten Konnektion ein Summensignal aus den durch den arteriellen und den venösen Patientenanschluss zum Drucksensor gelangenden Druckpulsen vom Herz des Patienten darstellen.

Weiterhin kann bei dem Verfahren vorgesehen sein bzw. die Steuerung der erfindungsgemäßen Vorrichtung oder Blutbehandlungsvorrichtung so ausgestaltet sein, dass bereits bei einer Abweichung der Amplitude der Druckpulse von weniger als 90% und bevorzugt weniger als 75% von einer bei einer korrekten Konnektion vorliegenden Amplitude der Druckpulse auf eine Diskonnektion geschlossen wird. Damit wird berücksichtigt, dass bei dem erfindungsgemäßen Summensignal bei der Diskonnektion nur der arteriellen Zugangs oder nur des venösen Zugangs weiterhin Signale vom Herzen über den anderen Drucksensor gelangen und die Amplitude daher trotz Diskonnektion nicht auf null abfällt.

Insbesondere kann bei einer Veränderung des Signals auf eine Diskonnektion geschlossen werden, obwohl das Signal weiterhin Druckpulse vom Herz des Patienten enthält, deren Amplitude 10% und bevorzugt 25% von einer bei einer korrekten Konnektion vorliegenden Amplitude beträgt.

Erfindungsgemäß kann weiterhin die Impellerpumpe oder Zentrifugalpumpe zwischen dem Drucksensor und dem venösen Patientenzugang angeordnet ist, und/oder wobei der Drucksensor in der venösen Tropfkammer angeordnet ist.

Weiterhin kann vorgesehen sein, wobei durch den Vergleich der am extrakorporalen Blutkreislauf gemessenen Amplituden der Druckpulse vom Herz des Patienten mit mindestens einem Grenzwert oder Referenzverlauf sowohl eine venöse als auch eine arterielle Diskonnektion erkannt werden, und/oder wobei durch die Auswertung der Drucksignale eines einzigen Drucksensors sowohl eine venöse als auch eine arterielle Diskonnektion erkannt werden.

Weiterhin kann erfindungsgemäß keine Auftrennung der Drucksignale in Frequenzkomponenten und/oder keine Unterdrückung oder Filterung von Frequenzkomponenten erfolgen, d.h. der erfindungsgemäße Vergleich mit einem Grenzwert bzw. Referenzverlauf aufgrund eines Signals erfolgen, welches nicht in dieser Form aufbereitet wurde.

Weiterhin kann die Erfassung eine Nadel-Diskonnektion ohne Berücksichtigung der Form, Frequenz und/oder absoluten Höhe des gemessenen Signals erfolgen.

Weiterhin kann vorgesehen sein, dass eine Nadel-Diskonnektion spätestens nach 5, bevorzugt nach 3, besonders bevorzugt nach 1,5 Perioden der Druckpulse vom Herz des Patienten detektiert wird und/oder nach einer unzulässigen Veränderung der Druckamplitude, welche länger als 5, bevorzugt länger als 3, besonders bevorzugt länger als 1,5 Perioden der Druckpulse vom Herz des Patienten andauert, auf eine Nadel-Diskonnektion geschlossen wird und/oder wobei nach einer unzulässigen Veränderung der Druckamplitude, welche länger als 5, bevorzugt länger als 3, besonders bevorzugt länger als 1,5 Sekunden andauert, auf eine Nadel-Diskonnektion geschlossen wird.

Bevorzugt erfolgt das oben dargestellte Vorgehen durch die Steuerung der erfindungsgemäßen Blutbehandlungsvorrichtung automatisch.

Weiterhin kann die Steuer- und Recheneinheit dabei konfiguriert und/oder programmiert sein, um das oben dargestellte Vorgehen automatisch durchzuführen. Weitere Merkmale, Einzelheiten und Vorteile der Erfindung werden anhand eines Ausführungsbeispiels im Folgenden anhand einer Figur näher erläutert.

Die einzige Figur zeigt den gemessenen Druckverlauf des venösen und arteriellen Druckverlaufs an einem mittels einer Impeller-Blutpumpe betriebenen extrakorporalen Blutkreislaufs bei Anzeigen des Ereignisses einer venösen Nadeldiskonnektion.

Das Verfahren gemäß der vorliegenden Erfindung ist gemäß dem hier dargestellten Ausführungsbeispiel in einer extrakorporalen Blutbehandlungsvorrichtung realisiert. Bei der extrakorporalen Blutbehandlungsvorrichtung kann es sich beispielsweise um eine bekannte Hämodialysevorrichtung handeln, wie sie beispielsweise in der DE 10 2009 060 668 A1 beschrieben ist.

Auf die Beschreibung der dort beispielhaft aufgeführten Hämodialysevorrichtung als typische Blutbehandlungsvorrichtung zum Einsatz der vorliegenden Erfindung wird verwiesen. Die extrakorporale Blutbehandlungsvorrichtung der Erfindung weist insbesondere eine Steuer- und Recheneinheit und einen maschinenseitigen Antrieb für eine Impeller-Blutpumpe auf. Die Impeller-Blutpumpe besteht aus einem Gehäuse mit Impeller und ist vorzugsweise Bestandteil des extrakorporalen Blutschlauchsatzes, der besonders vorteilhaft als Disposable-Blutkassette ausgeführt ist, wobei der extrakorporale Blutschlauchsatz zur Ankopplung an die extrakorporale Blutbehandlungsvorrichtung konfiguriert ist.

Weiterhin weist die Blutbehandlungsmaschine mindestens einen Drucksensor auf, der zum Ankoppeln an eine Druckmessstelle des extrakorporalen Blutschlauchsatzes konfiguriert ist.

Der Drucksensor und die Impeller-Blutpumpe stehen mit der Steuer- und Recheneinheit in Verbindung. Vorteilhaft kann eine drahtlose Übertragung am Disposable-Blutschlauchsatz als Verbindung zu der Steuer- und Recheneinheit vorgesehen sein. Derartige drahtlose Verbindungen sind von aus dem Anwendungsfall integrierter RFID-Drucksensoren mit Disposable-Blutschlauchsatz bekannt.

Die Steuer- und Recheneinheit weist einen Datenspeicher auf, in dem ein Computerprogramm abgespeichert ist. Der Programmcode des Computerprogramms ist programmiert, um die Drucksignale des mindestens einen Drucksensors auszuwerten. Hier werden insbesondere die am extrakorporalen Blutkreislauf gemessenen Amplituden der Druckpulse vom Herz des Patienten mit mindestens einem Grenzwert oder Referenzverlauf verglichen. Der Grenzwert oder Referenzverlauf wird aufgrund einer initialen Messung im störungsfrei betriebenen extrakorporalen Blutkreislauf zuvor festgelegt. In diesem Fall ist die Steuer- und Recheneinheit so ausgelegt, dass der Grenzwert oder Referenzverlauf, der zuvor im Datenspeicher abgespeichert wurde, mit den aktuellen Messwerten verglichen wird. Beim Auftreten einer unzulässigen Abweichung der gemessenen Amplitude der Druckpulse vom Herz des Patienten wird auf einen fehlerhaften Gefäßzugang, im vorliegenden Fall auf eine venöse Nadeldiskonnektion geschlossen.

Im Rahmen der vorliegenden Erfindung ist es nicht mehr notwendig, zumindest eine arterielle Druckmessstelle und eine venöse Druckmessstelle am extrakorporalen Blutschlauchsatz vorzusehen. Für die Ausführung der Erfindung reicht es vollständig aus, mindestens einen einzigen Drucksensor am extrakorporalen Blutschlauchsatz anzuordnen, da sich im Falle einer arteriellen und/oder venösen Nadeldiskonnektion überall im extrakorporalen Blutkreislauf die Amplitude des gemessenen Herzdruckpulses ändert. Das gemessene Signal stellt dabei ein Summensignal der vom Herz erzeugten, über den venösen und den arteriellen Zugang in den extrakorporalen Blutschlauchsatz übertragenen Signale. In dem Falle einer Diskonnektion nur der arteriellen Nadel oder nur der venösen Nadel würde sich die gemessene Amplitude der Druckpulse vom Herz des Patienten damit verändern, ohne auf Null abzufallen. Im Falle einer Diskonnektion der arteriellen Nadel und der venösen Nadel würde die gemessene Amplitude der Druckpulse vom Herz des Patienten auf Null abfallen. Die Zuverlässigkeit der Erkennung einer Nadeldiskonnektion hängt dabei natürlich entscheidend von den gewählten Grenzwerten oder Referenzverläufen ab.

Anhand der Figur kann ein typischer zu erfassender Signalverlauf für eine Nadel-diskonnektion erläutert werden.

Hier sind drei Kurvenverläufe aufgezeichnet. Der obere Verlauf zeigt den venösen Druckverlauf, der untere Verlauf den arteriellen Druckverlauf. Diese Druckverläufe sind in einem extrakorporalen Blutkreislauf gemessen, der mittels einer Impeller-Blutpumpe betrieben wird. In der Mitte ist der Verlauf der durch Herzkontraktion verursachten Druckpulswellen des Patienten dargestellt. Es wird deutlich, dass nach der venösen Nadeldiskonnektion, d. h. zu einem Zeitpunkt t=25 sek der Amplitudenverlauf der gemessenen venösen und arteriellen Druckverläufe stark abnehmen. Diese Veränderung kann als Fehler, d. h. als Nadeldiskonnektion erfasst werden. Der parallel ermittelte Verlauf der durch die Herzkontraktion verursachten Druckpulswellen des Patienten entsprechend der mittleren Kurve in der Figur ist anderweitig gemessen worden und ist hier nur zum Vergleich in derselben Grafik dargestellt. In der erfindungsgemäßen Vorrichtung wird diese Kurve üblicherweise nicht aufgenommen. Der Vergleich mit dieser Kurve zeigt jedoch, dass die Pulse des venösen und arteriellen Druckverlaufs synchron mit denen der Herzpulse verlaufen und dass daher keine nennenswerten anderen Störsignale vorhanden sind. Dies ist nur aufgrund der Verwendung der Impeller-Blutpumpe oder Zentrifugal-Blutpumpe möglich.

Im Rahmen der vorliegenden Erfindung wird insbesondere folgendes Messprinzip eingesetzt:
Die Druckpulskurve der Herzpulse wird im extrakorporalen Blutkreislauf (EBK) gemessen. Da das System erfindungsgemäß ohne okkludierende Komponenten, insbesondere ohne peristaltische Pumpen auskommt, ist es als offenes System ausgeprägt, d.h. die Druckpulse des Herzens gelangen durch beide Patientenzugänge in den EBK und überlagern sich dort. Es stellt sich somit ein Summensignal ein, welches nicht oder nur sehr wenig durch (Druck-)Aktuatoren des EBK selbst gestört ist. Durch das offene Systemkonzept kann der Drucksensor an jeder beliebigen Stelle im System angeordnet sein. Beispielsweise kann ein Drucksensor in der venösen Tropfkammer verwendet werden.

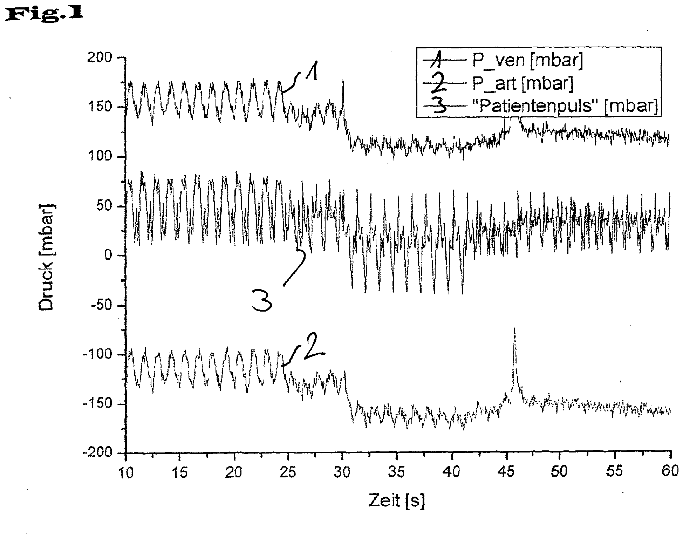

Nach Diskonnektion einer der beiden Patientenleitungen liegt am Drucksensor im EBK ein verändertes Signal vor, welches kein Summensignal mehr ist. Dadurch verändert sich die Druckamplitude (halbe Differenz der lokalen Minima und Maxima der Druckpulskurve) signifikant. Im Beispiel in Figur 1 verändert sich dieser Wert bei der bei ca. 25 s auftretenden Venösen Diskonnektion von ca. 40 mbar auf ca. 20 mbar, d.h. um ungefähr Faktor 2.

Weiterhin erzeugt die Impellerpumpe oder eine Zentrifugalpumpe anders als eine Peristaltikpumpe keine eigenen Druckpulse. Wegen der fehlenden Störsignale aus dem EBK kann das Summensignal aus den vom Herz des Patienten kommenden Signale daher direkt ausgewertet werden. Erfindungsgemäß ist keine Nachverarbeitung des Signals im Sinne einer Transformation in den Frequenzraum und Filtern der ungewünschten Signalanteile notwendig. Das System kommt ohne Fouriertransformation aus und somit muss kein kontinuierlich wiederkehrendes Signal vorliegen. Im wesentlichen kann erfindungsgemäß eine venöse Nadel-Diskonnektion bereits nach 1,5 Perioden detektiert werden.

Weiterhin ist die Detektion von der absoluten Veränderung der Signallage unabhängig, solange lokale Minima und Maxima erkennbar sind. Ebenso stellen Frequenzveränderungen, etwa bei schwankender Herzfrequenz, keine Beeinflussung des Systems dar.

Es ist ferner unerheblich, ob eine venöse oder eine arterielle Nadel-Diskonnektion vorliegt. Beide Ereignisse können auf dem gleichen Weg detektiert werden.

## Patentansprüche

1. Vorrichtung zur Überwachung einer extrakorporalen Blutbehandlungsvorrichtung mit einem extrakorporalen Blutkreislauf, der eine arterielle Blutleitung mit einem arteriellen Patientenanschluss und eine venöse Blutleitung mit einem venösen Patientenanschluss aufweist, wobei in der arteriellen oder venösen Blutleitung eine Pumpe zum Fördern von Blut im extrakorporalen Blutkreislauf angeordnet ist, wobei die Vorrichtung eine Überwachungsvorrichtung mit einer Steuer- und Recheneinheit aufweist, die programmiert und konfiguriert ist, eine arterielle und/oder venöse Nadeldiskonnektion zu erkennen,
**dadurch gekennzeichnet,**
**dass** die Pumpe eine Impellerpumpe oder eine Zentrifugalpumpe ist, wobei die Steuer- und Recheneinheit programmiert und konfiguriert ist, das folgende Verfahren durchzuführen:
- Messen mindestens einer ersten Druckamplitude und/oder eines ersten Verlaufs einer Druckamplitude als Referenzwert an mindestens einer Druckmessstelle des extrakorporalen Blutkreislaufs,
- Berechnen mindestens eines Druckgrenzwertes aufgrund der ersten als Referenzwert aufgenommenen Druckamplitude und/oder Festlegen eines Referenz-Druckverlaufs aufgrund des ersten Verlaufs der Druckamplitude,
- Messen mindesten einer zweiten Druckamplitude und/oder eines zweiten Verlaufs der Druckamplitude an der mindestens einen Druckmessstelle des extrakorporalen Blutkreislaufs,
- Vergleichen der zweiten Druckamplitude mit dem mindestens einen Druckgrenzwert und/oder Vergleichen des zweiten Verlaufs der Druckamplitude mit dem Referenz Druckverlauf und
- Stoppen oder Drosseln der Blutpumpe und/oder Schließen der venösen Schlauchklemme, falls eine unzulässige Abweichung von dem Referenzwert und/oder dem Referenz-Druckverlauf festgestellt wird.

2. Vorrichtung nach Anspruch 1, **dadurch gekennzeichnet, dass** die Steuer- und Recheneinheit so programmiert und konfiguriert ist, dass bei Feststellung einer unzulässigen Abweichung ein akustischer und/oder optischer und/oder taktiler Alarm ausgegeben wird.

3. Vorrichtung nach Anspruch 1 oder 2, **dadurch gekennzeichnet, dass** die Impeller-Blutpumpe oder die Zentrifugal-Blutpumpe derart angesteuert ist, dass ihre Drehzahl konstant ist.

4. Vorrichtung nach einem der Ansprüche 1 bis 3, **dadurch gekennzeichnet, dass** die Steuer- und Recheneinheit so programmiert und konfiguriert ist, dass die Druckpulse des Herzens aufgenommen werden.

5. Vorrichtung nach einem der vorangegangenen Ansprüche, wobei die Steuer-und Recheneinheit so programmiert und konfiguriert ist, dass die Amplitude der Druckpulse aus der Differenz der lokalen Minima und Maxima der Druckpulskurve ermittelt wird.

6. Vorrichtung nach einem der vorangegangenen Ansprüche, wobei die Steuer-und Recheneinheit so programmiert und konfiguriert ist, dass die Drucksignale des mindestens einen Drucksensors bei einer korrekten Konnektion ein Summensignal aus den durch den arteriellen und den venösen Patientenanschluss zum Drucksensor gelangenden Druckpulsen vom Herz des Patienten darstellen, und/oder dass bereits bei einer Abweichung der Amplitude der Druckpulse von weniger als 90% und bevorzugt weniger als 75% von einer bei einer korrekten Konnektion vorliegenden Amplitude der Druckpulse auf eine Diskonnektion geschlossen wird.

7. Vorrichtung nach einem der vorangegangenen Ansprüche, wobei die Impellerpumpe oder Zentrifugalpumpe zwischen dem Drucksensor und dem venösen Patientenzugang angeordnet ist, und/oder wobei der Drucksensor in der venösen Tropfkammer angeordnet ist.

8. Vorrichtung nach einem der vorangegangenen Ansprüche, wobei die Steuer-und Recheneinheit so programmiert und konfiguriert ist, dass durch den Vergleich der am extrakorporalen Blutkreislauf gemessenen Amplituden der Druckpulse vom Herz des Patienten mit mindestens einem Grenzwert oder Referenzverlauf sowohl eine venöse als auch eine arterielle Diskonnektion erkannt werden, und/oder wobei die Steuer- und Recheneinheit so programmiert und konfiguriert ist, dass durch die Auswertung der Drucksignale eines einzigen Drucksensors sowohl eine venöse als auch eine arterielle Diskonnektion erkannt werden.

9. Vorrichtung nach einem der vorangegangenen Ansprüche, wobei die Steuer-und Recheneinheit so programmiert und konfiguriert ist, dass keine Auftrennung der Drucksignale in Frequenzkomponenten und/oder keine Unterdrückung oder Filterung von Frequenzkomponenten erfolgt und/oder wobei eine Nadel-Diskonnektion spätestens nach 5, bevorzugt nach 3, besonders bevorzugt nach 1,5 Perioden der Druckpulse vom Herz des Patienten detektiert wird und/oder nach einer unzulässigen Veränderung der Druckamplitude, welche länger als 5, bevorzugt länger als 3, besonders bevorzugt länger als 1,5 Perioden der Druckpulse vom Herz des Patienten andauert, auf eine Nadel-Diskonnektion geschlossen wird und/oder wobei nach einer unzulässigen Veränderung der Druckamplitude, welche länger als 5, bevorzugt länger als 3, besonders bevorzugt länger als 1,5 Sekunden andauert, auf eine Nadel-Diskonnektion geschlossen wird.

10. Blutbehandlungsvorrichtung zum Betrieb mit einem extrakorporalen Blutkreislauf, der eine arterielle Blutleitung mit einem arteriellen Patientenanschluss und eine venöse Blutleitung mit einem venösen Patientenanschluss aufweist, wobei in der arteriellen oder venösen Blutleitung eine Impellerpumpe oder eine Zentrifugalpumpe zum Fördern von Blut im extrakorporalen Blutkreislauf angeordnet ist, mit einer Steuerung, die programmiert und konfiguriert ist, eine Nadeldiskonnektion zu erkennen, wobei die Steuerung programmiert ist, um die Drucksignale mindestens eines Drucksensors auszuwerten,
**dadurch gekennzeichnet,**
**dass** die Steuerung so programmiert und konfiguriert ist, dass die am extrakorporalen Blutkreislauf gemessenen Amplituden der Druckpulse vom Herz des Patienten mit mindestens einem Grenzwert oder Referenzverlauf verglichen werden, wobei die Drucksignale des mindestens einen Drucksensors bei einer korrekten Konnektion ein Summensignal aus den durch den arteriellen und den venösen Patientenanschluss zum Drucksensor gelangenden Druckpulsen vom Herz des Patienten darstellen, und dass bereits bei einer Abweichung der Amplitude der Druckpulse von weniger als 90% und bevorzugt weniger als 75% von einer bei einer korrekten Konnektion vorliegenden Amplitude der Druckpulse auf eine Diskonnektion geschlossen wird.

11. Blutbehandlungsvorrichtung nach Anspruch 10, wobei die Steuerung so programmiert und konfiguriert ist, dass der Grenzwert oder Referenzverlauf aufgrund einer initialen Messung im störungsfrei betriebenen extrakorporalen Blutkreislauf zuvor festgelegt wird, und/oder wobei die Steuer- und Recheneinheit so ausgelegt ist, dass der Grenzwert oder Referenzverlauf, der zuvor im Datenspeicher abgespeichert wurde, mit den aktuellen Messwerten verglichen wird, wobei beim Auftreten einer unzulässigen Abweichung der gemessenen Amplitude der Druckpulse vom Herz des Patienten auf einen fehlerhaften Gefäßzugang geschlossen wird.

12. Blutbehandlungsvorrichtung nach einem der vorangegangenen Ansprüche, wobei die Steuerung so programmiert und konfiguriert ist, dass die Amplitude der Druckpulse aus der Differenz der lokalen Minima und Maxima der Druckpulskurve ermittelt wird.

13. Blutbehandlungsvorrichtung nach einem der vorangegangenen Ansprüche, wobei die Impellerpumpe oder Zentrifugalpumpe zwischen dem Drucksensor und dem venösen Patientenzugang angeordnet ist, und/oder wobei der Drucksensor in der venösen Tropfkammer angeordnet ist.

14. Blutbehandlungsvorrichtung nach einem der vorangegangenen Ansprüche, wobei die Steuerung so programmiert und konfiguriert ist, dass durch den Vergleich der am extrakorporalen Blutkreislauf gemessenen Amplituden der Druckpulse vom Herz des Patienten mit mindestens einem Grenzwert oder Referenzverlauf sowohl eine venöse als auch eine arterielle Diskonnektion erkannt werden, und/oder wobei die Steuerung so programmiert und konfiguriert ist, dass durch die Auswertung der Drucksignale eines einzigen Drucksensors sowohl eine venöse als auch eine arterielle Diskonnektion erkannt werden.

15. Blutbehandlungsvorrichtung nach einem der vorangegangenen Ansprüche, wobei die Steuerung so programmiert und konfiguriert ist, dass keine Auftrennung der Drucksignale in Frequenzkomponenten und/oder keine Unterdrückung oder Filterung von Frequenzkomponenten erfolgt und/oder eine Nadel-Diskonnektion spätestens nach 5, bevorzugt nach 3, besonders bevorzugt nach 1,5 Perioden der Druckpulse vom Herz des Patienten detektiert wird und/oder nach einer unzulässigen Veränderung der Druckamplitude, welche länger als 5, bevorzugt länger als 3, besonders bevorzugt länger als 1,5 Perioden der Druckpulse vom Herz des Patienten andauert, auf eine Nadel-Diskonnektion geschlossen wird und/oder nach einer unzulässigen Veränderung der Druckamplitude, welche länger als 5, bevorzugt länger als 3, besonders bevorzugt länger als 1,5 Sekunden andauert, auf eine Nadel-Diskonnektion geschlossen wird.

## Claims

1. Device for monitoring an extracorporeal blood treatment device comprising an extracorporeal blood circuit which comprises an arterial blood line having an arterial patient connection, and a venous blood line having a venous patient connection, wherein a pump for conveying blood in the extracorporeal blood circuit is arranged in the arterial or venous blood line, wherein the device comprises a monitoring device having a control and arithmetic unit which is programmed and configured to identify an arterial and/or venous needle disconnection,
**characterised in that**
the pump is an impeller pump or a centrifugal pump, wherein the control and arithmetic unit is programmed and configured to carry out the following method:
- Measuring at least one first pressure amplitude and/or a first curve of a pressure amplitude as a reference value at at least one pressure measuring point of the extracorporeal blood circuit,
- Calculating at least one pressure threshold value on the basis of the first pressure amplitude assumed as a reference value, and/or setting a reference pressure curve on the basis of the first curve of the pressure amplitude,
- Measuring at least one second pressure amplitude and/or a second curve of the pressure amplitude at the at least one pressure measuring point of the extracorporeal blood circuit,
- Comparing the second pressure amplitude with the at least one pressure threshold value, and/or comparing the second curve of the pressure amplitude with the reference pressure curve, and
- Stopping or throttling the blood pump, and/or closing the venous tubing clamp if an inadmissible deviation from the reference value and/or the reference pressure curve is identified.

2. Device according to claim 1, **characterised in that** the control and arithmetic unit is programmed and configured such that, upon identification of an inadmissible deviation, an acoustic and/or visual and/or tactile alarm is issued.

3. Device according to either claim 1 or claim 2, **characterised in that** the impeller blood pump or the centrifugal blood pump is controlled such that the speed thereof is constant.

4. Device according to any of claims 1 to 3, **characterised in that** the control and arithmetic unit is programmed and configured such that the pressure pulses of the heart are recorded.

5. Device according to any of the preceding claims, wherein the control and arithmetic unit is programmed and configured such that the amplitude of the pressure pulses is determined from the difference between the local minima and maxima of the pressure pulse curve.

6. Device according to any of the preceding claims, wherein the control and arithmetic unit is programmed and configured such that, in the case of a correct connection, the pressure signals of the at least one pressure sensor represent a composite signal made up of the pressure pulses from the patient's heart that reach the pressure sensor via the arterial and the venous patient connection, and/or such that a disconnection is already concluded at a deviation of the amplitude of the pressure pulses of less than 90%, and preferably less than 75%, from an amplitude of the pressure pulses existing in the case of a correct connection.

7. Device according to any of the preceding claims, wherein the impeller pump or centrifugal pump is arranged between the pressure sensor and the venous patient access, and/or wherein the pressure sensor is arranged in the venous drip chamber.

8. Device according to any of the preceding claims, wherein the control and arithmetic unit is programmed and configured such that both a venous and an arterial disconnection are identified from the comparison of the amplitudes of the pressure pulses from the patient's heart measured at the extracorporeal blood circuit with at least one threshold value or reference curve, and/or wherein the control and arithmetic unit is programmed and configured such that both a venous and an arterial disconnection are identified from the evaluation of the pressure signals of a single pressure sensor.

9. Device according to any of the preceding claims, wherein the control and arithmetic unit is programmed and configured such that the pressure signals are not separated into frequency components, and/or frequency components are not suppressed or filtered, and/or wherein a needle disconnection is detected at the latest after 5, preferably after 3, particularly preferably after 1.5 periods of the pressure pulses from the patient's heart and/or a needle disconnection is concluded after an inadmissible change in the pressure amplitude which lasts longer than 5, preferably longer than 3, particularly preferably longer than 1.5, periods of the pressure pulses of the patient's heart, and/or wherein a needle disconnection is concluded after an inadmissible change in the pressure amplitude which lasts longer than 5, preferably longer than 3, particularly preferably longer than 1.5, seconds.

10. Blood treatment device for operation with an extracorporeal blood circuit comprising an arterial blood line having an arterial patient connection, and a venous blood line having a venous patient connection, wherein an impeller pump or a centrifugal pump for conveying blood in the extracorporeal blood circuit is arranged in the arterial or venous blood line, comprising a controller which is programmed and configured for identifying a needle disconnection, wherein the controller is programmed to evaluate the pressure signals of at least one pressure sensor,
**characterised in that**
the controller is programmed and configured such that the amplitudes of the pressure pulses from the patient's heart that are measured at the extracorporeal blood circuit are compared with at least one threshold value or reference curve, wherein, in the case of a correct connection, the pressure signals of the at least one pressure sensor represent a composite signal made up of the pressure pulses from the patient's heart that reach the pressure sensor via the arterial and the venous patient connection, and/or such that a disconnection is already concluded at a deviation of the amplitude of the pressure pulses of less than 90%, and preferably less than 75%, from an amplitude of the pressure pulses existing in the case of a correct connection.

11. Blood treatment device according to claim 10, wherein the controller is programmed and configured such that the threshold value or reference curve is specified in advance, on the basis of an initial measurement in the extracorporeal blood circuit that is operated in a fault-free manner, and/or wherein the control and arithmetic unit is designed such that the threshold value or reference curve that was previously stored in the data memory is compared with the current measured values, wherein an incorrect vascular access is concluded in the event of the occurrence of an inadmissible deviation of the measured amplitude of the pressure pulses from the patient's heart.

12. Blood treatment device according to any of the preceding claims, wherein the controller is programmed and configured such that the amplitude of the pressure pulses is determined from the difference of the local minima and maxima of the pressure pulse curve.

13. Blood treatment device according to any of the preceding claims, wherein the impeller pump or centrifugal pump is arranged between the pressure sensor and the venous patient access, and/or wherein the pressure sensor is arranged in the venous drip chamber.

14. Blood treatment device according to any of the preceding claims, wherein the controller is programmed and configured such that both a venous and an arterial disconnection can be identified by comparing the amplitudes of the pressure pulses from the patient's heart, measured at the extracorporeal blood circuit, with at least one threshold value or reference curve, and/or wherein the controller is programmed and configured such that both a venous and an arterial disconnection can be identified by evaluating the pressure signals of a single pressure sensor.

15. Blood treatment device according to any of the preceding claims, wherein the controller is programmed and configured such that there is no separation of the pressure signals into frequency components and/or no suppression or filtering of frequency components, and/or a needle disconnection is detected at the latest after 5, preferably after 3, particularly preferably after 1.5 periods of the pressure pulses from the patient's heart, and/or a needle disconnection is concluded following an inadmissible change in the pressure amplitude which lasts longer than 5, preferably longer than 3, particularly preferably longer than 1.5 periods of the pressure pulses from the patient's heart, and/or a needle disconnection is concluded following an inadmissible change in the pressure amplitude which lasts longer than 5, preferably longer than 3, particularly preferably longer than 1.5 seconds.

## Revendications

1. Dispositif de surveillance d'un dispositif de traitement du sang extracorporel avec un circuit sanguin extracorporel, qui présente un conduit sanguin artériel avec un raccordement au patient artériel et un conduit sanguin veineux avec un raccordement au patient veineux, dans lequel une pompe servant à refouler du sang dans le circuit sanguin extracorporel est disposée dans le conduit sanguin artériel ou veineux, dans lequel le dispositif présente un dispositif de surveillance avec une unité de commande et de calcul, qui est programmée et configurée pour identifier une déconnexion d'aiguille artérielle et/ou veineuse,
**caractérisé en ce que**
la pompe est une pompe à rotor ou une pompe centrifuge, dans lequel l'unité de commande et de calcul est programmée et configurée pour mettre en œuvre le procédé suivant :
- de mesure au moins d'une première amplitude de pression et/ou d'une première évolution d'une amplitude de pression en tant que valeur de référence au niveau d'au moins un emplacement de mesure de pression du circuit sanguin extracorporel,
- de calcul au moins d'une valeur limite de pression en raison de la première amplitude de pression relevée en tant que valeur de référence et/ou de fixation d'une évolution de pression de référence en raison de la première évolution de l'amplitude de pression,
- de mesure au moins d'une deuxième amplitude de pression et/ou d'une deuxième évolution de l'amplitude de pression au niveau de l'au moins un emplacement de mesure de pression du circuit sanguin extracorporel,
- de comparaison de la deuxième amplitude de pression avec l'au moins une valeur limite de pression et/ou de comparaison de la deuxième évolution de l'amplitude de pression à l'évolution de pression de référence, et
- d'arrêt ou d'étranglement de la pompe à sang et/ou de fermeture du collier de serrage pour tuyau veineux si un écart non admissible par rapport à la valeur de référence et/ou à l'évolution de pression de référence est constaté.

2. Dispositif selon la revendication 1, **caractérisé en ce que** l'unité de commande et de calcul est programmée et configurée de telle sorte qu'en cas de constatation d'un écart non admissible, une alarme acoustique et/ou optique et/ou tactile est émise.

3. Dispositif selon la revendication 1 ou 2, **caractérisé en ce que** la pompe à sang à rotor ou la pompe à sang centrifuge sont pilotées de telle manière que leur vitesse de rotation est constante.

4. Dispositif selon l'une quelconque des revendications 1 à 3, **caractérisé en ce que** l'unité de commande et de calcul est programmée et configurée de telle sorte que les impulsions de pression du cœur sont relevées.

5. Dispositif selon l'une quelconque des revendications précédentes, dans lequel l'unité de commande et de calcul est programmée et configurée de telle sorte que l'amplitude des impulsions de pression est déterminée à partir de la différence entre les minima et maxima locaux de la courbe d'impulsion de pression.

6. Dispositif selon l'une quelconque des revendications précédentes, dans lequel l'unité de commande et de calcul est programmée et configurée de telle sorte que les signaux de pression de l'au moins un capteur de pression constituent, dans le cas d'une connexion correcte, un signal cumulé composé des impulsions de pression du cœur du patient parvenant au capteur de pression par le raccordement au patient artériel et le raccordement au patient veineux, et/ou qu'une déconnexion est déduite déjà dans le cas d'un écart de l'amplitude des impulsions de pression inférieur à 90 % et de manière préférée inférieur à 75 % par rapport à une amplitude des impulsions de pression régnant dans le cas d'une connexion en bonne et due forme.

7. Dispositif selon l'une quelconque des revendications précédentes, dans lequel la pompe à rotor ou la pompe centrifuge est disposée entre le capteur de pression et l'accès au patient veineux, et/ou dans lequel le capteur de pression est disposé dans une chambre de goutte à goutte veineuse.

8. Dispositif selon l'une quelconque des revendications précédentes, dans lequel l'unité de commande et de calcul est programmée et configurée de telle sorte qu'à la fois une déconnexion veineuse et une déconnexion artérielle sont identifiées par la comparaison des amplitudes mesurées au niveau du circuit sanguin extracorporel des impulsions de pression du cœur du patient à au moins une valeur de limite ou une évolution de référence, et/ou dans lequel l'unité de commande et de calcul est programmée et configurée de telle sorte qu'à la fois une déconnexion veineuse et une déconnexion artérielle sont identifiées par l'évaluation des signaux de pression d'un unique capteur de pression.

9. Dispositif selon l'une quelconque des revendications précédentes, dans lequel l'unité de commande et de calcul est programmée et configurée de telle sorte qu'une séparation des signaux de pression dans des composantes de fréquence et/ou aucune suppression ni aucun filtrage des composantes de fréquence n'ont lieu et/ou dans lequel une déconnexion d'aiguille est détectée au plus tard après 5, de manière préférée 3, de manière particulièrement préférée après 1,5 périodes des impulsions de pression du cœur du patient et/ou une déconnexion d'aiguille est déduite après une modification non admissible de l'amplitude de pression, dont la durée est supérieure à 5, de manière préférée supérieure à 3, de manière particulièrement préférée supérieure à 1,5 périodes des impulsions de pression du cœur du patient, et/ou dans lequel une déconnexion d'aiguille est déduite après une modification non admissible de l'amplitude de pression, dont la durée est supérieure à 5, de manière préférée supérieure à 3, de manière particulièrement préférée supérieure à 1,5 secondes.

10. Dispositif de traitement du sang destiné à fonctionner avec un circuit sanguin extracorporel, qui présente un conduit sanguin artériel avec un raccordement au patient artériel et un conduit sanguin veineux avec un raccordement au patient veineux, dans lequel une pompe à rotor ou une pompe centrifuge servant à refouler du sang dans le circuit sanguin extracorporel est disposée dans le conduit sanguin artériel ou veineux, avec une commande, qui est programmée et configurée pour identifier une déconnexion d'aiguille, dans lequel la commande est programmée pour évaluer les signaux de pression au moins d'un capteur de pression,
**caractérisé en ce**
**que** la commande est programmée et configurée de telle sorte que les amplitudes, mesurée au niveau du circuit sanguin extracorporel, des impulsions de pression du cœur du patient sont comparées à au moins une valeur limite ou une évolution de référence, dans lequel les signaux de pression de l'au moins un capteur de pression constituent, dans le cas d'une connexion en bonne et due forme, un signal cumulé composé des impulsions de pression, parvenant au capteur de pression par le raccord au patient artériel et le raccord au patient veineux, du cœur du patient, et qu'une déconnexion est déduite déjà dans le cas d'un écart de l'amplitude des impulsions de pression inférieur à 90 % et de manière préférée inférieur à 75 % d'une amplitude, régnant dans le cas d'une connexion en bonne et due forme, des impulsions de pression.

11. Dispositif de traitement du sang selon la revendication 10, dans lequel la commande est programmée et configurée de telle sorte que la valeur limite ou l'évolution de référence est fixée au préalable en raison d'une mesure initiale dans le circuit sanguin extracorporel fonctionnant sans problème, et/ou dans lequel l'unité de commande et de calcul est configurée de telle sorte que la valeur limite ou l'évolution de référence, qui a été sauvegardée au préalable dans la mémoire de données, est comparée aux valeurs de mesure instantanées, dans lequel dans le cas de l'apparition d'un écart non admissible de l'amplitude mesurée des impulsions de pression du cœur du patient, un accès vasculaire erroné est déduit.

12. Dispositif de traitement du sang selon l'une quelconque des revendications précédentes, dans lequel la commande est programmée et configurée de telle sorte que l'amplitude des impulsions de pression est déterminée à partir de la différence des minima et maxima locaux de la courbe d'impulsions de pression.

13. Dispositif de traitement du sang selon l'une quelconque des revendications précédentes, dans lequel la pompe à rotor ou la pompe centrifuge est disposée entre le capteur de pression et l'accès au patient veineux, et/ou dans lequel le capteur de pression est disposé dans la chambre goutte à goutte veineuse.

14. Dispositif de traitement du sang selon l'une quelconque des revendications précédentes, dans lequel la commande est programmée et configurée de telle sorte qu'à la fois une déconnexion veineuse et une déconnexion artérielle sont identifiées par la comparaison des amplitudes, mesurées au niveau du circuit sanguin extracorporel, des impulsions de pression du cœur du patient à au moins une valeur limite ou une évolution de référence, et/ou dans lequel la commande est programmée et configurée de telle sorte qu'une déconnexion veineuse et une déconnexion artérielle sont identifiées par l'évaluation des signaux de pression d'un unique capteur de pression.

15. Dispositif de traitement du sang selon l'une quelconque des revendications précédentes, dans lequel la commande est programmée et configurée de telle sorte qu'aucune séparation des signaux de pression n'est effectuée dans des composantes de fréquence et aucune suppression ou aucun filtrage de composantes de fréquence n'a lieu et/ou une déconnexion d'aiguille est détectée au plus tard après, 5, de manière préférée après 3, de manière particulièrement préférée après 1,5 périodes des impulsions de pression du cœur du patient et/ou une déconnexion d'aiguille est déduite après une modification non admissible de l'amplitude de pression, dont la durée est supérieure à 5, de manière préférée est supérieure à 3, de manière particulièrement préférée est supérieure à 1,5 périodes des impulsions de pression du cœur du patient et/ou une déconnexion d'aiguille est déduite après une modification non admissible de l'amplitude de pression, dont la durée est supérieure à 5, de manière préférée supérieure à 3, de manière particulièrement préférée supérieure à 1,5 secondes.
